(19) Europäisches Patentamt
European Patent Office
Office européen des brevets

(11) **EP 4 395 879 B1**

(12) **EUROPEAN PATENT SPECIFICATION**

(45) Date of publication and mention
of the grant of the patent:
**13.08.2025 Bulletin 2025/33**

(21) Application number: **22762110.9**

(22) Date of filing: **17.08.2022**

(51) International Patent Classification (IPC):
*A61N 1/08* (2006.01)     *A61N 1/36* (2006.01)
*G01R 31/50* (2020.01)     *A61B 5/276* (2021.01)
*A61B 5/294* (2021.01)     *A61B 5/053* (2021.01)

(52) Cooperative Patent Classification (CPC):
**A61N 1/08; A61B 5/276; A61B 5/294;
A61N 1/36128;** A61N 1/36062; A61N 1/3614;
A61N 1/36142; A61N 2001/083; G01R 31/67

(86) International application number:
**PCT/EP2022/072967**

(87) International publication number:
**WO 2023/030889 (09.03.2023 Gazette 2023/10)**

(54) **METHOD TO CHECK A MEDICAL DEVICE AND METHOD OF OPERATING THE SAME**

VERFAHREN ZUR ÜBERPRÜFUNG EINER MEDIZINISCHEN VORRICHTUNG UND VERFAHREN ZUM BETRIEB DAVON

PROCÉDÉ DE CONTRÔLE D'UN DISPOSITIF MÉDICAL ET SON PROCÉDÉ DE FONCTIONNEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.09.2021  US 202163240081 P
13.10.2021  EP 21202404**

(43) Date of publication of application:
**10.07.2024  Bulletin 2024/28**

(73) Proprietor: **BIOTRONIK SE & Co. KG
12359 Berlin (DE)**

(72) Inventors:
• **ALVES, Luis Genehr
Lake Oswego, Oregon 97034 (US)**
• **RIAHI, Pamela Shamsie Victoria
Portland, Oregon 97232 (US)**

(74) Representative: **Biotronik Corporate Services SE
Corporate Intellectual Property
Sieversufer 7-9
12359 Berlin (DE)**

(56) References cited:
**US-A1- 2006 122 653      US-A1- 2010 106 206
US-A1- 2019 076 659      US-A1- 2020 132 434
US-A1- 2020 146 581**

## Description

The invention refers to a method to check a medical device such as a medical device used for spinal cord stimulation (SCS) or a medical device for gathering information such as vital health parameters, cardiac activity, bio-chemical activity, electrical activity in the brain, gastric activity or other physiological data. The medical devices typically comprise electrodes arranged on a lead body that are electrically linked to a pulse generator which provides therapy signals to said electrodes. The electrodes may be used for stimulation as well as for sensing. In addition, the invention refers to a method of operating such medical devices, to the medical device itself, to a system comprising the medical device and a remote computer, and to a computer program product for executing said methods.

Depending on the use of the medical device various implant locations exist. For spinal cord stimulation at least one lead body of the medical device such as a surgical lead or percutaneous leads is commonly implanted within the epidural space of the spinal cord. The at least one lead body of said medical device comprises electrodes electrically linked to a pulse generator such as an implantable pulse generator to provide neurostimulation. The pulse generator comprises at least one port having connections which are electrically and mechanically connected to the electrodes, for example by a plug connector. By said electrode stimulation nociception is influenced and chronic pains are alleviated. Thus, electrical signals of the nervous system, which are transmitted via the spinal cord and registered by the brain, are influenced by electrical signals from the electrodes of the medical device in such a way that the sensation of pain, for example, is perceived to be reduced.

A critical factor of SCS therapy as in any other electrode stimulation therapy is therefore the correct positioning of the electrodes as well as the controlling of said electrodes. Accordingly, the therapy efficiency and pain relief in SCS are substantially decreased if, for example, an electrode is energized which is different from the one that is actually meant, for example by an incorrect electrical electrode-pulse generator connection. Since the at least one lead body is implanted, however, to check the reason for an incorrect functioning of the medical device, methods such as medical imaging, paresthesia mapping, or other electrical tests are known. These methods, however, often require complex programming and/or a high amount of energy which might be critical for the longevity of the medical device. In addition, these methods are time-consuming and therefore increase the risk of undesired electrode stimulations. Further, the methods may be able to determine why a medical device is not functioning as it should, but at the same time require a subsequent surgical procedure with additional risks to correct the positioning or the incorrectly linked connections of the electrodes of the medical device.

A method for determining the relative positioning of lead bodies is disclosed in US 2020/0132434 A1. This document describes the determination of a relative longitudinal offset between pairs of electrodes of the lead bodies, in particular on the basis of measured impedance values.

However, as mentioned above, sometimes electrodes are linked incorrectly, so that, for example, electrodes are electrically linked to incorrect ports of the pulse generator or to incorrect connections of the port of the pulse generator. In this context, 'incorrect' means that the pulse generator sends an electrical signal to a connection and thereby to an interconnected electrode or to an interconnected group of electrodes that was intended for a different electrode or group of electrodes.

The importance of a correct connection of electrodes was already identified in US 2020/0093566 A1. This document describes the determination and association of unique identification codes to a connector or to an electrode group lead, wherein an input channel of a control unit is associated to an electrode or electrode group lead based on the determined at least one of the unique identification codes. Further, it was identified that the set-up process of a medical device is time consuming since the connection corresponding to each electrode needs to be separately established and then verified for integrity.

US 2019/0076659 A1 describes a system and method for determination of connected neurostimulation leads. The types of electrode leads that are connected to an implantable medical device are determined based on electrical parameters that are measured at the electrodes that are positioned on the leads. The different types of known electrode leads have different physical electrode arrangements that impact the measured electrical parameters. Properties in the measured electrical parameters that are indicative of the physical arrangements of electrodes of known types of electrode leads are utilized to determine the types of leads that are connected to the implantable medical device.

Therefore, there is a need for a simple, reliable and cost effective method to check whether electrodes of a lead body of a medical device are correctly electrically linked to a pulse generator. In addition, there is a need for a method that, when identifying an incorrectly electrically linked electrode with a connection of a port of a pulse generator, allows correct provisioning of the electrode with electrical signals without surgical intervention or at least a correct functioning of the medical device without surgical procedure.

The above problem is solved by a method to check a medical device with the features of claim 1 and a method of operating a medical device with the features of claim 11. Further, the above problem is solved by a medical device with the features of claim 12 and a system comprising the medical device and a remote computer with the features of claim 14. Furthermore, the problem is solved by a computer program product according to claim 15.

The present invention is defined by the appended claims.

SUMMARY OF THE PRESENT DISCLOSURE

The described method to check a medical device, wherein said medical device comprises at least two electrode groups and a pulse generator, wherein each electrode group comprises at least two electrodes, wherein the at least two electrode groups are arranged on a surface of at least one lead body, wherein the pulse generator comprises at least one port, wherein the number of connections of the at least one port is equal to or greater than the number of electrodes of all electrode groups and form connection groups corresponding to the electrode groups, wherein the electrodes are electrically linked using at least one lead connector to the at least one port of the pulse generator, comprises the steps of:

(a) selecting pre-defined pairs of connections, wherein the connections of each pair are assigned to different connection groups, wherein at least one connection is varied in one pre-defined pair with regard to any other selected pair;

(b) measuring an impedance value between the connections of each of the selected pairs of connections; and

(c) checking whether the electrical links of the electrodes and the connections are correctly established based on determined pre-conditioned impedance values and/or the measured impedance values of the selected pairs of connections, wherein the pre-conditioned impedance value is determined for each of the selected pairs of connections such that it provides a pre-conditioning of the respective measured impedance value for attenuating unwanted noise.

The at least one lead body of the medical device for which the inventive method is intended is implanted in the patient's body and enables stimulation and/or sensing of bodily signals to reduce, for example, chronic pain. In case the medical device is used for SCS, the at least one lead body is typically implanted in the epidural fat and configured to provide electrostimulation using the electrodes provided on and across the surface of the at least one lead body. The electrostimulation is directed towards the neural elements in the spinal cord and configured to influence nociception and alleviate chronic pain.

The medical device comprises at least one lead body. The lead body may have an elongated shape with the electrodes arranged on the outer surface of the lead body and along the length of the lead body. The electrodes are electrically insulated from one another and may have an extension of a few millimeters. Further, the electrodes are arranged in electrode groups, wherein each lead body comprises at least one electrode group with at least two electrodes. Thus, in order to make proper use of the inventive method and to check a medical device at least two electrode groups each comprising at least two electrodes are needed. Consequently, there may be provided only one lead body with said at least two electrode groups each comprising at least two electrodes or the medical device may have, for example, more than one lead body, wherein each lead body comprises at least one electrode group with at least two electrodes. If the medical device is used for SCS, the at least one lead body may comprise e.g. at least 16 electrodes.

For stimulation or sensing, the electrodes are electrically linked and mechanically connected to the pulse generator, wherein said pulse generator may be an implantable pulse generator (IPG). The pulse generator is configured to deliver electrical signals to the electrodes, thereby providing electrostimulation, and/or to sense/receive electrical signals using said electrodes. Accordingly, the pulse generator controls the operation of the electrodes and therefore may be referred to as control unit, as well. For SCS, the pulse generator may be implanted in a distal location such as the lower back and programmed to analyze electrical signals received via electrodes and to deliver electrical signals through the electrodes to stimulate neurons in the spinal cord. Therefore, the pulse generator comprises at least one port comprising a pre-defined number of connections, wherein each connection of a port is intended to be electrically linked to exactly one electrode. Hence, the number of connections of the at least one port is equal to or greater than the number of electrodes of all electrode groups. Further, since the electrodes are arranged in electrode groups such groups may also be defined as connection groups in the port. The connections of the at least one port are electrically linked to the electrodes and the electrode groups respectively using at least one lead connector.

Based on said aforementioned configuration of a medical device, the inventive method comprises at least three steps in order to check whether the electrical links of the electrodes and the connections of the port are correctly established. To do so, in step (a) the method selects automatically pre-defined pairs of connections (i,j). Each pair of connections comprises two connections, wherein each connection is assigned to a different connection group. Thus, each selected pre-defined pair of connections comprises one connection i of a first connection group and one connection j of a second connection group, wherein the first connection group and the second connection group are different. Further pairs of connections are identified varying at least one of the two connections. As each of the connections is associated with one electrode, i.e. is electrically linked to one electrode, pairs of connections may be selected so that each possible pairing of electrodes is conducted.

In step (b) impedance values $Z_{i,j}$ are automatically measured between each of said selected pairs of connections. If necessary, said measured impedance values are then automatically pre-conditioned to attenuate unwanted noise, e.g. electrode-tissue interface impedance contribution to the measured impedance values, and to determine a pre-conditioned impedance value $Z''_{i,j}$ for each of the selected pairs of connections as indicated below in detail. The smaller the distance between two electrodes of a pair of connections arranged on the surface of the lead body, the smaller the measured impedance value or the determined pre-conditioned impedance value. Conversely, the greater the distance between two electrodes of a pair of connections arranged on the surface of the lead body, the greater the measured impedance value or the determined pre-conditioned impedance value. The measured impedance values and/or the determined pre-conditioned impedance values of the pairs of connection allow to estimate the relative distance between respective electrodes linked to said connections. The measured impedance values or the determined pre-conditioned impedance values may then be compared with a set of target impedance values or target pre-conditioned impedance values for the selected pairs of connections and it may be determined from these determined data whether the electrodes are correctly linked to the connections or not. Accordingly, more generally taken, in step (c) of the inventive method it is automatically checked whether the electrical links of the electrodes and the connections are correctly established based on the measured impedance values and/or on determined pre-conditioned impedance values of the selected pairs of connections. This may be achieved by measurement refinement via an error estimation and subtraction process or by peak detection and validation or by pattern correlation estimation.

In order to attenuate unwanted noise (e.g. electrode-tissue interface impedance contributions to the measured impedances) from the measured impedance values $Z_{i,j}$ of the pairs of connections (i,j), wherein i is a connection of the first group of connections and j is a connection of a second group of connections, the pre-conditioned impedance values $Z''_{i,j}$ are calculated as follows. At first, automatically for each connection j of the second group of connections an average impedance $\ddot{Z}_j$ (e.g. arithmetic mean value) from the measured impedances of the connection pairs (i,j) comprising the connection j are calculated and the average impedance $\ddot{Z}_j$ is subtracted from the measured impedances $Z_{i,j}$ of the pairs of connections (i,j) comprising the connection j and processed impedances $Z'_{i,j}$ are obtained. Additionally, automatically calculating for each connection i of the first group of connections an average processed impedance $\overline{Z'_i}$ (e.g. arithmetic mean value) from said processed impedances $Z'_{i,j}$ of the pairs of connections (i,j) comprising the connection i of the first group of connections and subtracting the average processed impedance $(\overline{Z'_i})$ from the processed impedances $Z'_{i,j}$ of the pairs of connections (i,j) comprising the connection i of the first group of connections to obtain the pre-conditioned impedances $Z''_{i,j}$. If there is only one measured impedance value $Z_{i,j}$ for each connection i or for each connection j of all measured pairs of connections (i,j), the above average value $\overline{Z}_j$ and $\overline{Z'_i}$ is set to 0 as

For example, a medical device may be assumed comprising one elongated lead body with two electrode groups each comprising 8 electrodes, i.e. 16 electrodes in total, wherein said electrodes groups are parallelly arranged, and wherein each electrode is electrically linked to a connection of a port of the pulse generator, the electrode groups are reflected in the form of connection groups. If now the impedance between a pair of connections is measured, wherein the two connections are assumed to be linked to the most distal electrodes are used for the measurement, wherein one connection refers to the one connection group and the other connection refers to the other connection group, a first impedance value is determined. If now one connection of the aforementioned pair of connections is varied, wherein it is assumed that said varied connection now refers to the second most distal electrode, a second impedance value and a respective pre-conditioned impedance value is determined. Since the distance between the two most distal electrodes should in principle be smaller than the distance between the most distal electrode of one group and the second most distal electrode of the other group, the second impedance value measured between the second pair of connections should be greater than the first measured impedance value between the first pair of connections. If not, this may indicate that one of the electrical links of the electrodes and the connections are not correctly established.

'Distal' is used here to specify a direction along the elongated lead body which points from the port of the pulse generator. In contrast, 'proximal' is used to indicate a direction along the elongated lead body pointing towards the port of the pulse generator.

The impedances between the connections may be measured in such a way that one connection of each pair of connections is kept constant throughout all pairs of connections and the other connection is varied so that the impedance value between the constant connection and each other connection of the other group is measured. In this case a uniform pattern of pre-conditioned impedance values may be obtained if the electrodes are correctly electrically linked to the connections and if the values are plotted in a diagram.

'Correctly established' means that one specific connection of the port of the pulse generator is actually connected to the electrode of the lead body to which the pulse generator expects to be connected, so that the pulse generator delivers electrical signals to an electrode that are intended for this electrode or the received electrical signals from one specific connection can be assigned to an electrode that is known with regard to its location at the lead body. If an electrode is identified which is incorrectly electrically linked to a connection of the port of the pulse generator, there are basically three options. First, if there is one electrode which is incorrectly electrically linked to a connection of the pulse generator, there should be at least one more electrode incorrectly electrically linked. Thus, the pulse generator may identify all electrodes which are incorrectly electrically linked and may then determine the location of the incorrectly linked electrodes at the at least one lead body and adapt the electrode control by the pulse generator to the different wiring. Second, the pulse generator may identify the incorrectly electrically linked electrodes using the checking method and may then disable the link to the connection to these incorrectly electrically linked electrodes so that it is no longer possible to receive or deliver faulty signals to/from these electrodes. Third, the electrical link of the electrodes and the connections may be specifically changed at the port.

In one example, the method further comprises the step of labeling the connections of the at least one port, wherein the connections of one connection group are entirely labeled in a pre-defined way, for example consecutively numbered, wherein the labeling of all connections is provided such that it reflects the distance of each electrode of one electrode group to each electrode of a different electrode group if each electrode is correctly electrically linked to the respective connection. The connections may be labeled using numbers or letters, any combination of these or the like.

Assuming the above-mentioned example of a medical device comprising one elongated lead body with two electrode groups each comprising 8 electrodes, i.e. 16 electrodes in total, wherein said electrodes groups are parallelly arranged, the connections of one connection group referring to one electrode group are labeled. The first connection group may be numbered from 1 to 8 and the second connection group may be numbered from 9 to 16. Alternatively, the connections of the first connection group may be labeled from A to H and the second connection group may be labeled from I to P. Generally speaking, this results in a pair of connections $(i,j)$, wherein $i$ represents the label of one of the connections of the first connection group and j the label of the other connection of the second connection group. Accordingly, the impedance values according to step (b) may be $Z_{i,j}$ and the pre-conditioned impedance values according to step (c) with regard a pair of connections $(i,j)$ may be $Z''_{i,j}$. The measured impedance values for all possible pairs of connections may therefore be expressed in the following matrix according to Table 1:

*Table 1 - impedance values of a medical device comprising two connection groups each comprising 8 connections*

| | | Connection group 1 | | | | | | | |
|---|---|---|---|---|---|---|---|---|---|
| | Connection # | 1 | 2 | 3 | 4 | 5 | 6 | 7 | 8 |
| Connection group 2 | 9 | Z1,9 | Z2,9 | Z3,9 | Z4,9 | Z5,9 | Z6,9 | Z7,9 | Z8,9 |
| | 10 | Z1,10 | Z2,10 | Z3,10 | Z4,10 | Z5,10 | Z6,10 | Z7,10 | Z8,10 |
| | 11 | Z1,11 | Z2,11 | Z3,11 | Z4,11 | Z5,11 | Z6,11 | Z7,11 | Z8,11 |
| | 12 | Z1,12 | Z2,12 | Z3,12 | Z4,12 | Z5,12 | Z6,12 | Z7,12 | Z8,12 |
| | 13 | Z1,13 | Z2,13 | Z3,13 | Z4,13 | Z5,13 | Z6,13 | Z7,13 | Z8,13 |
| | 14 | Z1,14 | Z2,14 | Z3,14 | Z4,14 | Z5,14 | Z6,14 | Z7,14 | Z8,14 |
| | 15 | Z1,15 | Z2,15 | Z3,15 | Z4,15 | Z5,15 | Z6,15 | Z7,15 | Z8,15 |
| | 16 | Z1,16 | Z2,16 | Z3,16 | Z4,16 | Z5,16 | Z6,16 | Z7,16 | Z8,16 |

If, for example, the connections are labeled by numbers, so that connections which are assumed to be assigned to the most distal electrodes of the respective electrode groups are assigned, for example, the numbers 1 and 9, and the connections are labeled consecutively from here, so that the connections which are assumed to be assigned to the second most distal electrodes are labeled 2 and 10, the label of the connections reflects the distance of each electrode of one electrode group to each electrode of a another electrode group. Hence, the distance between electrodes referring to connections 1 and 9 is smaller than the distance between electrodes referring to connections 1 and 10. More generally, from the labelling of the connections the distance of correctly linked electrodes may directly be derived.

In one example of the disclosure, the method further comprises the step of assigning each measured impedance value and/or each pre-conditioned impedance value of one pair of labeled connections to one category of a pre-defined set of categories, and wherein the category of one pair of labeled connections is determined from the two labels of the one pair of connections. In one example, since the labeling of each pair of connections reflects the distance between corresponding electrodes of said pair, if correctly electrically linked, the pairs of connections reflecting the same distance between respective electrodes may be assigned to the same category. For example, connection pairs linked to opposite electrodes of different groups may be assigned to category 0, whereas connection pairs linked to electrodes with the maximum electrode distance with regard to above example of 8 electrodes and two electrode groups may be assigned to category +7 and -7, respectively. Other category types are possible, as well.

Assuming Table 1, the measured impedance values may be categorized as shown in Table 2 below.

Table 2 - impedance values according to Table 1 assigned to categories

| Category # | | | | | | | | | | | | | | |
|---|---|---|---|---|---|---|---|---|---|---|---|---|---|---|
| -7 | -6 | -5 | -4 | -3 | -2 | -1 | 0 | 1 | 2 | 3 | 4 | 5 | 6 | 7 |
|  |  |  |  |  |  |  | Z1,9 | Z1,10 | Z1,11 | Z1,12 | Z1,13 | Z1,14 | Z1,15 | Z1,16 |
|  |  |  |  |  |  | Z2,9 | Z2,10 | Z2,11 | Z2,12 | Z2,13 | Z2,14 | Z2,15 | Z2,16 |  |
|  |  |  |  |  | Z3,9 | Z3,10 | Z3,11 | Z3,12 | Z3,13 | Z3,14 | Z3,15 | Z3,16 |  |  |
|  |  |  |  | Z4,9 | Z4,10 | Z4,11 | Z4,12 | Z4,13 | Z4,14 | Z4,15 | Z4,16 |  |  |  |
|  |  |  | Z5,9 | Z5,10 | Z5,11 | Z5,12 | Z5,13 | Z5,14 | Z5,15 | Z5,16 |  |  |  |  |
|  |  | Z6,9 | Z6,10 | Z6,11 | Z6,12 | Z6,13 | Z6,14 | Z6,15 | Z6,16 |  |  |  |  |  |
|  | Z7,9 | Z7,10 | Z7,11 | Z7,12 | Z7,13 | Z7,14 | Z7,15 | Z7,16 |  |  |  |  |  |  |
| Z8,9 | Z8,10 | Z8,11 | Z8,12 | Z8,13 | Z8,14 | Z8,15 | Z8,16 |  |  |  |  |  |  |  |

In the above example, pre-conditioned impedance values $Z1,15$ and $Z2,16$ both assigned to category 6 correspond to the pairs of connections C1 and C15 and C2 and C16 respectively.

In one example, not for all possible pairs of connections as defined above the pre-conditioned impedance value needs to be determined but for a characteristic group. As one example, in step (a) the pairs of connections are selected such that at least one measured impedance value and/or determined pre-conditioned impedance value is assigned to each category. Further, the pairs of connections may be selected such that at least one pre-conditioned impedance value is determined for each connection. By determining at least one pre-conditioned impedance value for each connection, it is assured that each connection is included in the check whether the electrical links of the electrodes and the connections are correctly established.

**In** a further example, the method comprises the step of calculating an average value from all measured impedance values and/or from all determined pre-conditioned impedance values of the respective category. By calculating an average value for each category, measurement inaccuracies may be compensated. The average may be an arithmetic, geometric or harmonic mean or a median value of the measured impedance values and/or the determined pre-conditioned impedance values of the respective category. Referring to the example of Table 2, an average value for category 4 may be calculated from the impedance values $Z1,13$, $Z2,14$, $Z3,15$ and $Z4,16$. An average value for the pre-conditioned impedance values $Z1,14$, $Z2,15$ and $Z3,16$ may be calculated for category 5. Further, an average value of category 4 may be named $\overline{Z}4$, wherein an average value for category 5 may be named $\overline{Z}5$. Following Table 2, Table 3 corresponding to the average values from all pre-conditioned impedance values of the respective category is shown below:

*Table 3 - average value from all measured impedance values or all pre-conditioned impedance values of the respective category according to Table 2*

| Category # | | | | | | | | | | | | | | |
|---|---|---|---|---|---|---|---|---|---|---|---|---|---|---|
| -7 | -6 | -5 | -4 | -3 | -2 | -1 | 0 | 1 | 2 | 3 | 4 | 5 | 6 | 7 |
| $\overline{Z}$-7 | $\overline{Z}$-6 | $\overline{Z}$-5 | $\overline{Z}$-4 | $\overline{Z}$-3 | $\overline{Z}$-2 | $\overline{Z}$-1 | $\overline{Z}0$ | $\overline{Z}1$ | $\overline{Z}2$ | $\overline{Z}3$ | $\overline{Z}4$ | $\overline{Z}5$ | $\overline{Z}6$ | $\overline{Z}7$ |

Since in Table 2 for categories -7 and 7 only one pre-conditioned impedance value was listed, the average value of $\overline{Z}$-7 is $Z8,9$ and the average value of $\overline{Z}7$ is $Z1,16$.

In one example, the method further comprises the step of plotting said determined average impedance values and/or said average pre-conditioned impedance values and/or the measured impedance values and/or the determined pre-conditioned impedance values over the respective category in a diagram and analyzing a profile of a graph formed by said values over all categories in said diagram. This means that if one stays with the example according to Tables 2 and 3, either the values of Table 2 and/or of Table 3 will be plotted over the respective category. The analysis of the profile of the graph may comprise the examination of characteristic points of the plotted graph, for example the examination of extreme values. Alternatively or additionally, the deviation of the determined profile of the graph from a pre-defined target profile may be determined, for example by a least-squares analysis.

Furthermore, the analysis may be provided directly by the pulse generator or the pulse generator may be configured to transmit the measured impedance values to a remote computer which will then execute at least one of the above steps. The analysis may be triggered manually by an health care practitioner (HCP) and/or may be provided automatically, for example, after implantation and/or each activation of the medical device.

In one example, the method further comprises the step of comparing each pre-conditioned impedance value to a respective pre-defined target value for the respective pair of connections and/or the step of comparing each average pre-conditioned impedance value to a respective pre-defined target average value for the respective category. The target values may be pre-defined based on experience values or model calculations. Based on the deviation the method decides whether the electrical connections are correctly established. In one example, based on the deviation amount, it may be determined which electrical connections not correctly established and which connections are - if applicable - reversed or whether some of the connections are fully lost.

In one example, the type of lead body is identified based on said comparison and/or on said profile of the graph in said diagram. In general, there may be two types of lead bodies of an SCS device, so-called surgical leads and percutaneous leads. When a medical device is implanted with a surgical lead, there is only one lead body electrically linked to an implanted pulse generator, with all electrodes disposed on the surface of the lead body as previously described in connection with Tables 1 to 3. However, when a medical device is implanted with percutaneous leads, there are at least two lead bodies electrically separately or together linked to the implanted pulse generator, with electrodes disposed on the surface of each of the at least two lead bodies. The above and below explained method may be applied independently of the type of lead body based on the amount of the pre- impedance values as this value is proportional to the distance of the electrodes.

If, for example, the medical device comprises two percutaneous leads, the lead bodies may be shifted relative to each other so that, if one refers to above example, the electrodes of the connections 1 and 9 are directly opposite to each other but the electrodes of the connections 1 and 10 have the smallest distance. Analogously, the electrodes of connections 2 and 11 are opposite. Analogously, it may be derived from the impedance values that they indicate a relative far distance of the electrodes. If this distance pattern of pre-conditioned impedance values is measured throughout all pairs of connections, it is concluded that the lead body type is percutaneous leads.

In one aspect of the present disclosure, the method comprises the step of determining the one connection of the respective connection group electrically linked to the most distal electrode of the respective electrode group based on said comparison and/or on said profile of the graph in said diagram. This is derived from the fact, that for the connection with most distal electrode a number of different impedance values is measured for all pairs of connections having the most distal electrodes, wherein the number is the number of electrodes on the other group of electrodes. The different impedance values for pairs of connections with the most distal electrodes have the same sign. In contrast, if the connection with another electrode, the impedance values switch sign if the impedance values for all pairs of connection with the another electrode are measured. Determining the most distal electrode or positions of electrodes along the lead body in general forms the basis for successful alleviation of chronic pain.

In one example, the selected pre-defined pairs of connections are at most half of all possible pairs of connections. By reducing the number of pairs of connections and thus the number of impedance measurements, the method is be accelerated, energy and storage capacity is saved. The smaller amount of data makes the method less complex. The selected pre-defined pairs of connections may be such reduced that for each connection at least one impedance value is measured. The number of pairs of connections and thus the measurement values may also be reduced due to the fact that it is known which type of lead body is implanted.

The above problem is also solved by a method of operating a medical device, wherein the method comprises the steps of the checking method as explained above, and wherein the method further comprises the step of selecting an operation mode of the pulse generator based on the checking method, namely based on whether there is a connection identified that is incorrectly electrically linked to an electrode or not, and then operates according to the selected operation mode. Further, the operation mode may be selected, for example, based on the identified type of lead body. The operation mode may be selected based on the positioning of the electrodes, e.g. based on the identification of the most distal electrode. The operation mode may define the electrical signal values delivered to the electrodes by the pulse generator and, for example, the time point and signal sequence.

Further, the above object is solved by a medical device, for example for spinal cord stimulation, wherein said medical device comprises at least two electrode groups and a pulse generator, wherein each electrode group comprises at least two electrodes, wherein the at least two electrode groups are arranged on a surface of at least one lead body, wherein the pulse generator comprises at least one port, wherein the number of connections of the at least one port is equal to or greater than the number of electrodes of all electrode groups and form connection groups corresponding to the electrode groups, wherein the electrodes are electrically linked using at least one lead connector to the at least one port of the pulse generator, wherein the medical device is configured to execute the method steps explained in the above description.

In one example, the lead body of the medical device is a surgical lead or a percutaneous lead.

Furthermore, a system comprising the medical device according to the aforementioned description and a remote computer is disclosed. The remote computer of the system is at least temporarily connected to the medical device via a communication link, and wherein the remote computer is configured to execute a part of the method steps instead of the medical device. The remote computer may be operated by an HCP and may help to analyze and/or visualize the determined impedance values. The remote computer may be wired or wirelessly linked to the pulse generator and may be configured to process measured impedance values. For the communication link, the medical device and the remote computer may comprise a sender and receiver, respectively, or both a transceiver for sending and/or receiving communication signals.

Analogously, the present disclosure refers to a computer program product comprising instructions which, when executed, cause a processor to perform the steps of the methods according to the above-mentioned description. The computer program product may be a software routine, e.g. related to hardware support means within the IMD and/or the external device.

As already indicated before, the pulse generator is configured to control operation of each of the electrodes by delivering or by receiving electrical signals to or from the electrodes, wherein the pulse generator, inter alia, uses a computing processor in order to process, for example, received impedance measurement values.

Even if further processing of the impedance measurement values is not specified, the measurement values may also be processed with common mathematical methods known to those skilled in the art, for example, before the impedance values are plotted or before they are compared with target values. The further processing includes standard mathematical calculations and operations such as normalizing average pre-conditioned impedance values that may be collected in a vector and normalized to the interval [0,1].

The various features and advantages of the present invention may be more readily understood with reference to the following detailed description and the embodiments shown in the drawings. Herein schematically and exemplarily,

Fig. 1    depicts a first embodiment of a medical device according to the invention in a top view;

Fig. 2    depicts a second embodiment of a medical device in a top view;

Fig. 3    shows the embodiment of Fig. 1, with an open connection in a top view;

Fig. 4    illustrates a spinal cord cross section as well as a location of the lead body of the embodiment of Fig. 1 in the lower back of a patient;

Fig. 5    shows a plot of pre-conditioned impedance values as well as of average pre-conditioned impedance values of a medical device over respective categories in a diagram, wherein electrical links of electrodes and connections of said medical device are correctly established; and

Fig. 6    shows a plot according to Fig. 4 together with plotted pre-conditioned impedance values of the medical device with incorrectly established connections over respective categories in a diagram.

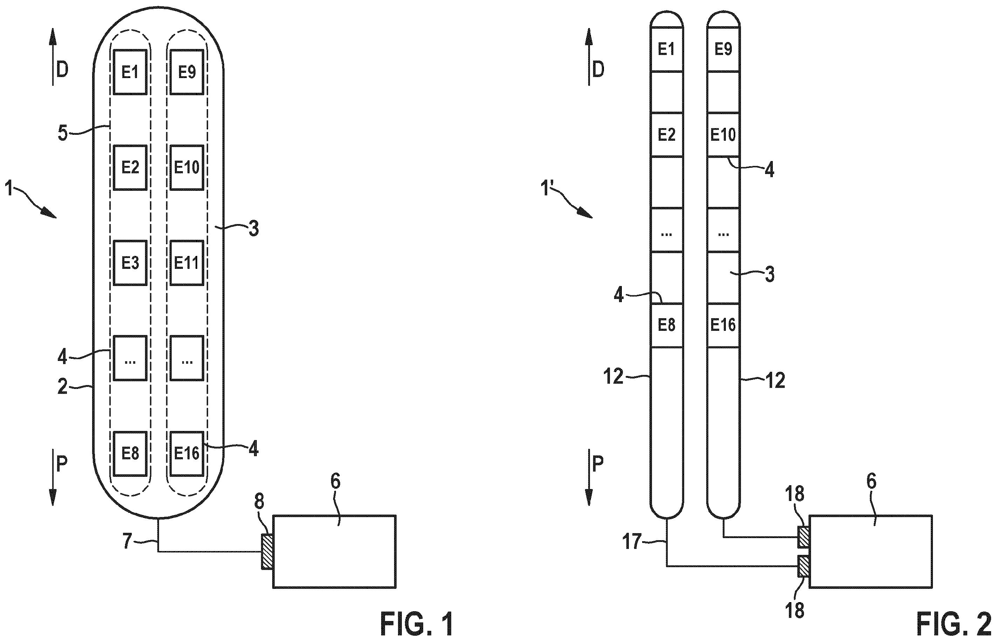

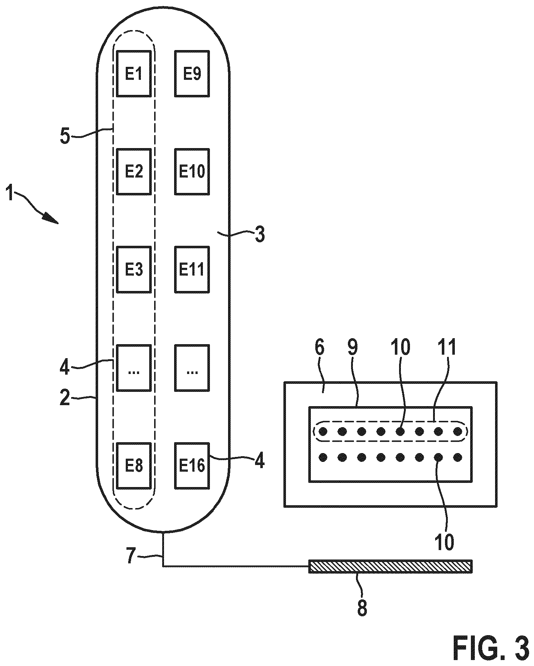

A medical device, namely an SCS device 1, forming an embodiment of the present invention comprises one surgical lead body 2 as shown in Fig. 1 and 3. The elongated lead body 2 comprises at its surface 3 sixteen electrodes 4 separated by insulating material (not shown). The sixteen electrodes 4 of the SCS devices 1 are labeled by numbers and the letter E, i.e. E1 to E16, in order to distinguish the electrode label from the reference numerals. The electrodes 4 with labels E4 to E7 and E12 to E15 are omitted in Fig. 1 and 3 and symbolized by the rectangles with three dots. The electrodes 4 with labels E1 to E8 and E9 to E16 each form one electrode group 5 indicated by dotted lines encircling said electrode groups. The left electrode group 5 is referred to as the first electrode group and the right electrode group 5 is referred to as the second electrode group in the following.

Each electrode 4 in Fig. 1 and 2 is electrically linked to a pulse generator 6, which may be an implantable pulse generator (IPG), via a connecting line 7 and a plug 8. The plug 8 is configured to be plugged into a port 9 in order to electrically link each electrode 4 to one connection 10 of the port 9. The pulse generator 6 receives electrical signals from or delivers electrical signals to the respective electrodes 4 via the plug 8 and the connecting line 7. In one embodiment, the pulse generator 6 may be configured to communicate with a remote computer.

As each electrode 4 is linked to (i.e. electrically connected with) one connection 10, the connections 10 may be labeled with the same numbers as the electrodes as explained above. Further, the connections 10 form two different groups of connections 11 as indicated by one dotted line in Fig. 3 encircling eight connections 10. Accordingly, the connections 10 may be labeled with the labels C1 to C8 and C9 to C16 and it is assumed as an example that a correct electrical linkage of the connections and the electrodes is established if each electrode is linked with the respective connection having the same number in its label, e.g. connection C1 to electrode E1, connection C2 to electrode E2 and so on.

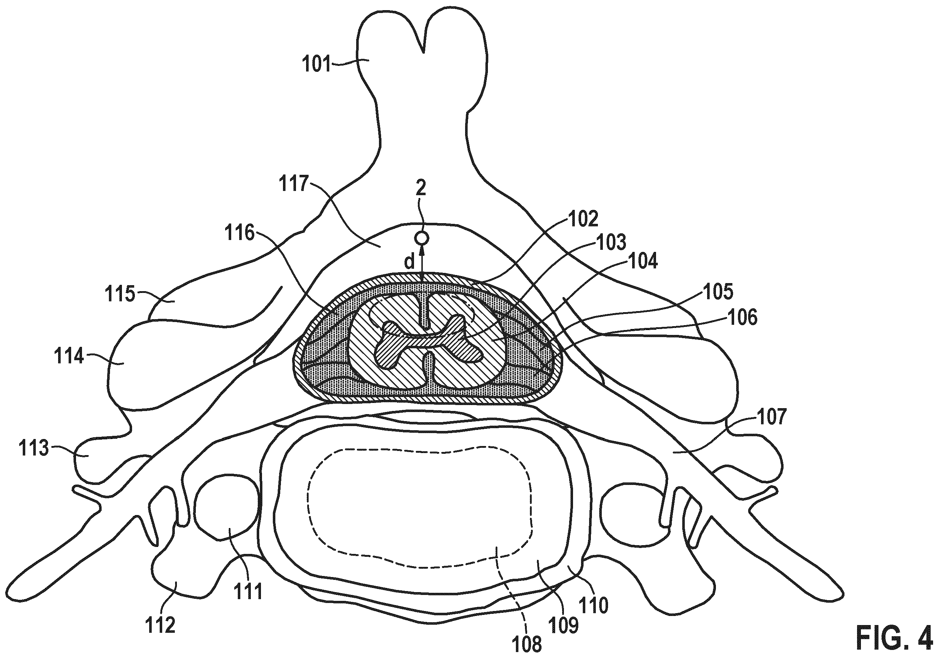

If the SCS device 1 is used for spinal cord stimulation (SCS) the lead body 2 is ideally placed the closest to the cerebrospinal fluid (CSF) 116 so that electrical current can flow through the CSF 116 in order to stimulate neurons into the spinal cord, i.e. white and grey matter 103, 104 (see Fig. 4). The white and grey matter 103, 104 is surrounded by the circulating CSF 116. Fig. 4 illustrates one possible application of SCS device 1 within the spinal cord cross section. The distance between the lead body 2 and the CSF 116 is illustrated by an arrow and marked 'd'. Further details of the spinal cord cross section are denoted with further reference numerals which are explained in the list of reference numerals below.

In the example of the shown SCS device 1, an epidural fat 117 between the electrodes 4 of the at least one lead body 2 and the CSF 116 drives the overall impedance between two electrodes. The extent to which it drives this impedance depends on the actual size of the epidural fat portion 117, i.e. the distance d between the electrodes 4 and the CSF 116. This variation can be captured by comparing the impedance between two adjacent electrodes and two distant electrodes on the same implanted device, for example, between electrodes labeled E1 and E9 and electrodes labeled E1 and E11.

If, however, the plug 8 is not correctly connected to the connection 10, i.e. incorrectly electrically linked, for example the electrode 4 with label E1 is electrically linked to the rightmost connection 10 of connection group 11 instead of being electrically linked to the leftmost connection 10, and electrode 4 with label E8 is electrically linked to the leftmost connection 10 instead of the rightmost connection 10 of this connection group 11, the pulse generator 6 addresses the electrode 4 with label E1 when the pulse generator 6 intends to address the electrode 4 with label E8 and vice versa. Hence, SCS therapy may, for example, be ineffective. In the same way, regarding signal recording, if the connections 10 are interchanged and connected not with the intended electrodes 4, the pulse generator 6 assigns received electrical signals to the wrong electrodes.

With the help of the checking method according to the invention, the pulse generator 6 is able to identify which electrode 4 is incorrectly electrically linked. The method according to the invention provides for the measurement of impedance values between the connections 10 and thus between the corresponding electrodes 4 electrically linked to said connections.

For example, just after implantation of the lead body 2 the pulse generator 6 checks the connections by measuring impedance values of selected pre-defined pairs of connections 10, wherein the connections 10 of each pair are assigned to different connection groups 11. The pairs are selected such that at least one connection 10 is varied in one pre-defined pair with regard to any other selected pair. For example, the pulse generator measures the number of 32 impedances of connection pairs (i,j), i.e. (C1, C9), (C1, C10), (C1, C11), ... , (C8, C16) and calculates the respective pre-conditioned impedance values $Z''_{i,j}$ of each of the selected pair of connections as explained above.

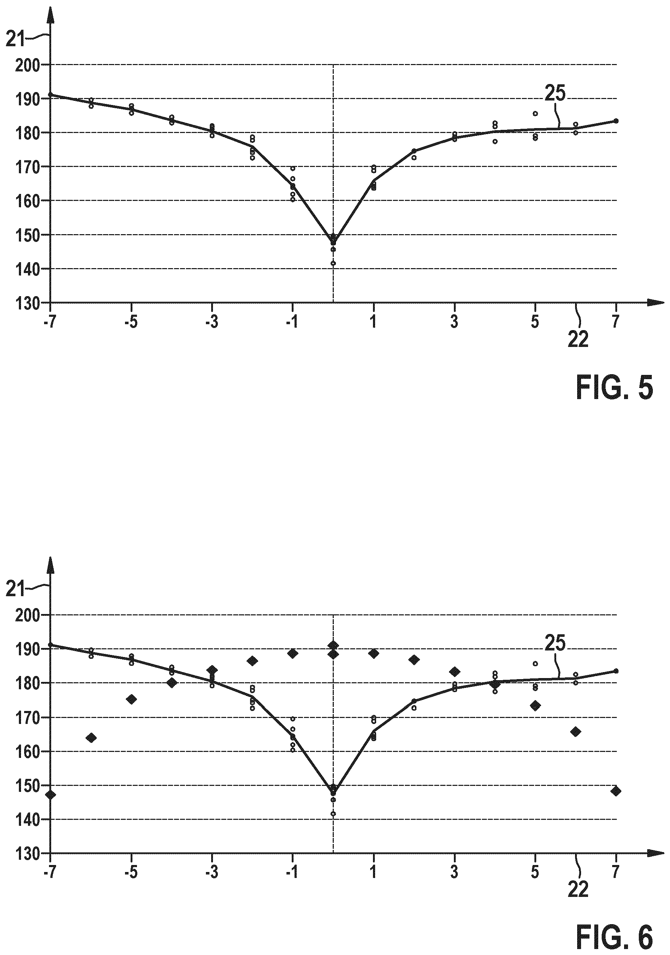

In the case that the connections 10 between the electrodes 4 and the pulse generator 6 are correctly established, the pre-conditioned impedance values $Z''_{i,j}$ will correspond to expected values, so-called target values or to the expected profile of a pre-defined graph. For example, in the diagram shown in Fig. 5 the pre-conditioned impedance values $Z''_{i,j}$ (see axis 21) are plotted over pre-defined categories (see axis 22).

For example, the connection pairs are assigned to categories which refer to the distance of electrodes that are (correctly) linked to these electrodes (also referred to as connection offset). As indicated in Table 2 above connection pairs (correctly) electrically connected to opposite electrodes of different groups may be assigned to category 0, whereas connection pairs (correctly) electrically connected to electrodes with maximum electrode distance with regard to current example of 8 electrodes and two electrode groups may be assigned to category +7 and -7, respectively. The remaining pre-conditioned impedance values are assigned to the corresponding category as indicated in Table 2. In the diagram shown in Fig. 5 the determined pre-conditioned impedance values (see small dots, ohms) are plotted over the category of the respective pair of connections. Further, the average value of all pre-conditioned impedance value $Z''_{i,j}$ of one category (e.g. arithmetic mean) is determined and a line 25 is drawn joining these average values of all categories.

Fig. 5 shows an example of correctly electrically linked electrodes, the pre-conditioned impedance values are lowest here for measured values of category 0, i.e. connections referring to opposite electrodes, for example connections with the labels C1 and C9, C2 and C10, C3 and C11, etc. linked to the electrodes E1 and E9, E2 and E10, E3 and E11, etc. The pair of connections with labels C1 and C16 linked to electrodes E1 and E16, on the other hand, wherein the respective pre-conditioned impedance values $Z''_{i,j}$ is plotted over category 7, has a significantly higher impedance values. The profile of the "V"-shaped graph (curve) 25 shown in Fig. 5 is characteristic of a surgical lead shown in Fig. 1 and 3 which is correctly connected.

In contrast, Fig. 6 shows the same diagram as depicted in Fig. 5 with the pre-conditioned impedance values $Z''_{i,j}$ of Fig. 5 and the average line 25 of Fig. 5 representing a correctly connected surgical lead of an SCS device. Pre-conditioned impedance values $Z''_{i,j}$ of another SCS device are plotted using rhombi over the respective category (see axis 22) for which the electrodes are incorrectly electrically linked to the connections C 1 to C16. In this example, the connections of the most distal and most proximal electrode of the second group of electrodes are swapped, i.e. the connections of electrodes E9 and E16. This means, that the pre-conditioned impedance value of the pair of connections C1 and C16 (1,16) which should have the greatest impedance is now smallest (see pre-conditioned impedance value at category -7) and the pre-conditioned impedance value for the pair of connections C1 and C9 (1,9) and C8 and C16 (8,16) is greatest due to this swap. Accordingly, the curve formed by the rhombi significantly differs from the usual (target) "V"-shaped profile 25.

Additionally, the largest pre-conditioned impedance value represents the impedance measurement between a pair of connections referring to electrodes having the greatest possible distance, i.e. one of the two electrodes is very likely the most distal electrode of the respective electrode group.

In conclusion, the above explained measurement of the impedance values between the connections 10 together with subsequent data processing and analyses is used to determine easily and cost-effectively whether the electrodes 4 of the at least one lead body 2 are correctly electrically linked to respective connections 10 of the pulse generator 6. Detailed analyses further allow statements to be made about the type of lead body 2 or which connection is linked to the most distal or proximal electrode of the respective group of electrodes in an easy and cost-effective way.

Fig. 2 illustrates a second embodiment of an SCS device 1' having two lead bodies 12 forming percutaneous leads. The location of the electrodes 4 provided at the surface 3 of the lead bodies 12 and their labeling corresponds to the surgical lead SCS device 1 shown in Fig. 1 and 3. Each lead body 12 is connected to a connecting line 17 and a plug 18 similarly to the first embodiment. Both plugs 18 are connected to a port of the pulse generator 6 thereby electrically linking/connecting the electrodes 4 to the respective connections of the pulse generator 6. It is apparent, that the plugs 18 can easily be swapped. The electrode of each lead body 12 form one group of eight electrodes. Accordingly, the pulse generator has sixteen connections forming two groups of connections. The method to check this embodiment of an SCS device is similar to the one described with regard to the embodiment of Fig. 1 and 3. However, the resulting profiles of graphs plotted over the category, for example, may be more complex because the lead bodies 12 carrying the electrodes 4 may be shifted with regard to each other.

The present invention is defined by the appended claims.

**List of reference numerals**

| | |
|---|---|
| 1, 1' | SCS device |
| 2, 12 | Lead body |
| 3 | Surface of the lead body |
| 4 | Electrode |
| 5 | Electrode group |
| 6 | (Implantable) pulse generator |
| 7, 17 | Connecting line |
| 8, 18 | Plug |
| 9 | Port |
| 10 | Connection |
| 11 | Connection group |
| 101 | Spinous process |
| 102 | Meninges |
| 103 | Gray matter |
| 104 | White matter |
| 105 | Dorsal root |
| 106 | Ventral root |
| 107 | Spinal nerve |
| 108 | Nucleus pulposus |
| 109 | Disc annulus |
| 110 | Vertebral body |
| 111 | Foramen transversium |
| 112 | Anterior tubercle of traverse process |
| 113 | Posterior tubercle of traverse process |
| 114 | Superior articular process |
| 115 | Inferior articular process |
| 116 | Cerebrospinal fluid |
| 117 | Epidural space of Foramen (filled with adipose tissue) |

**Claims**

1.   A method to check a medical device (1,1'), wherein said medical device (1,1') comprises at least two electrode groups (5) and a pulse generator (6), wherein each electrode group (5) comprises at least two electrodes (4), wherein the at least two electrode groups (5) are arranged on a surface (3) of at least one lead body (2), wherein the pulse generator (6) comprises at least one port (9), wherein the number of connections (10) of the at least one port (9) is equal to or greater than the number of electrodes (4) of all electrode groups (5) and form connection groups (11) corresponding to the electrode groups (5), wherein the electrodes (4) are electrically linked using at least one lead connector (8,18) to the at least one port (9) of the pulse generator (6), and wherein the method comprises the steps of:

      (a) selecting pre-defined pairs of connections, wherein the connections (10) of each pair are assigned to different connection groups (11), wherein at least one connection (10) is varied in one pre-defined pair with regard to any other selected pair;
      (b) measuring an impedance value between the connections (10) of each of the selected pairs of connections; and
      (c) checking whether the electrical links of the electrodes (4) and the connections (10) are correctly established based on determined pre-conditioned impedance values and/or the measured impedance values of the selected pairs of connections, wherein the pre-conditioned impedance value is determined for each of the selected pairs of connections such that it provides a pre-conditioning of the respective measured impedance value for attenuating unwanted noise.

2. The method according to claim 1, wherein the method further comprises the step of labeling the connections (10) of the at least one port (9), wherein the connections (10) of one connection group (11) are entirely labeled in a pre-defined way, for example consecutively numbered, wherein the labeling of all connections (10) is provided such that it reflects the distance of each electrode (4) of one electrode group (5) to each electrode of a different electrode group (5) if each electrode (4) is correctly electrically linked to the respective connection (10).

3. The method according to claim 2, wherein the method further comprises the step of assigning each measured impedance value and/or pre-conditioned impedance value of one pair of labeled connections to one category of a pre-defined set of categories, and wherein the category of one pair of labeled connections is determined from the two labels of the one pair of connections.

4. The method according to claim 3, wherein in step (a) the pairs of connections are selected such that at least one measured impedance value and/or determined pre-conditioned impedance value is assigned to each category.

5. The method according to claim 3 or 4, wherein the method further comprises the step of calculating an average value from all measured impedance values and/or determined pre-conditioned impedance values of the respective category.

6. The method according to any of the claims 3 to 5, wherein the method further comprises the step of plotting said determined average measured impedance values and/or average pre-conditioned impedance values and/or the measured impedance value and/or the determined pre-conditioned impedance values over the respective category in a diagram and analyzing a profile of a graph formed by said values over all categories in said diagram.

7. The method according to any of the previous claims, wherein the method further comprises the step of comparing each measured impedance value and/or pre-conditioned impedance value to a respective pre-defined target value for the respective pair of connections and/or the step of comparing each average measured impedance value and/or average pre-conditioned impedance value to a respective pre-defined target average value for the respective category.

8. The method according to claim 6 or 7, wherein the type of lead body (2,12) is identified based on said comparison and/or on said profile of the graph in said diagram.

9. The method according to claim 7 or 8, wherein the method further comprises the step of determining the one connection (10) of the respective connection group (11) electrically linked to the most distal electrode (4) of the respective electrode group (5) based on said comparison and/or on said profile of the graph in said diagram.

10. The method according to any of the previous claims, wherein the selected pre-defined pairs of connections are at most half of all possible pairs of connections.

11. A method of operating a medical device, wherein the method comprises the steps of the checking method according to any of the claims 1 to 10, and wherein the method further comprises the step of selecting an operation mode of the pulse generator (6) based on the checking method, for example based on the identified type of lead body (2) and then operates according to the selected operation mode.

12. A medical device (1,1'), for example for spinal cord stimulation, wherein said medical device (1,1') comprises at least two electrode groups (5) and a pulse generator (6), wherein each electrode group (5) comprises at least two electrodes (4), wherein the at least two electrode groups (5) are arranged on a surface (3) of at least one lead body (2,12), wherein the pulse generator (6) comprises at least one port (9), wherein the number of connections (10) of the at least one port (9) is equal to or greater than the number of electrodes (4) of all electrode groups (5) and form connection groups (11) corresponding to the electrode groups (5), wherein the electrodes (4) are electrically linked using at least one lead connector (8,18) to the at least one port (9) of the pulse generator (6), wherein the medical device (1,1') is configured to execute the method steps of any of claims 1 to 11.

13. The medical device according to claim 12, wherein the lead body (2,12) is a surgical lead or a percutaneous lead.

14. A system comprising the medical device (1,1') of claim 12 and a remote computer, wherein the remote computer is at least temporarily connected to the medical device (1,1') via a communication link, and wherein the remote computer is configured to execute a part of the method steps instead of the medical device (1,1').

**15.** A computer program product comprising instructions which, when executed, cause a processor of the medical device according to claim 12 to perform the steps of the methods according to any of claims 1 to 11.

**Patentansprüche**

**1.** Verfahren zum Prüfen eines medizinisches Geräts (1,1'), wobei genanntes medizinisches Gerät (1,1') mindestens zwei Elektrodengruppen (5) und einen Impulsgenerator (6) umfasst, wobei jede Elektrodengruppe (5) mindestens zwei Elektroden (4) umfasst, wobei die mindestens zwei Elektrodengruppen (5) auf einer Oberfläche (3) von mindestens einem Leitungskörper (2) angeordnet sind, wobei der Impulsgenerator (6) mindestens einen Port (9) umfasst, wobei die Anzahl der Anschlüsse (10) des mindestens einen Ports (9) gleich oder größer als die Anzahl der Elektroden (4) aller Elektrodengruppen (5) ist und Anschlussgruppen (11) bilden, die den Elektrodengruppen (5) entsprechen, wobei die Elektroden (4) elektrisch mittels mindestens eines Leitungsanschlusses (8,18) mit dem mindestens einen Port (9) des Impulsgenerators (6) verbunden sind, und wobei das Verfahren folgende Schritte umfasst:

(a) Auswahl vordefinierter Anschlusspaare, wobei die Anschlüsse (10) jedes Paares verschiedenen Anschlussgruppen (11) zugeordnet sind, wobei mindestens ein Anschluss (10) sich in einem vordefinierten Paar in Bezug auf ein anderes ausgewähltes Paar unterscheidet;

(b) Messen eines Impedanzwertes (10) zwischen den Anschlüssen von jedem der ausgewählten Anschluss-spaare; und

(c) Prüfen, ob die elektrischen Verbindungen der Elektroden (4) und die Anschlüsse (10) ordnungsgemäß hergestellt sind, auf Basis bestimmter vorkonditionierter Impedanzwerte und/oder der gemessenen Impedanzwerte der ausgewählten Anschlusspaare, wobei der vorkonditionierte Impedanzwert für jedes der ausgewählten Anschlusspaare so bestimmt wird, dass er eine Vorkonditionierung des jeweiligen gemessenen Impedanzwertes bereitstellt, um unerwünschtes Rauschen abzumildern.

**2.** Verfahren nach Anspruch 1, wobei das Verfahren ferner den Schritt umfasst, die Anschlüsse (10) des mindestens einen Ports (9) zu kennzeichnen, wobei die Anschlüsse (10) von einer Anschlussgruppe (11) vollständig in einer vordefinierten Art und Weise gekennzeichnet werden, zum Beispiel durchnummeriert werden, wobei das Kennzeichnen aller Anschlüsse (10) so vorgesehen ist, dass es den Abstand jeder Elektrode (4) einer Elektrodengruppe (5) zu jeder Elektrode einer anderen Elektrodengruppe (5) wiedergibt, wenn jede Elektrode (4) ordnungsgemäß elektrisch mit dem jeweiligen Anschluss (10) verbunden ist.

**3.** Verfahren nach Anspruch 2, wobei das Verfahren ferner den Schritt umfasst, jeden gemessenen Impedanzwert und/oder vorkonditionierten Impedanzwert eines Paares gekennzeichneter Anschlüsse einer Kategorie eines vordefinierten Satzes an Kategorien zuzuordnen, und wobei die Kategorie von einem Paar gekennzeichneter Anschlüsse aus den zwei Kennzeichnungen des einen Anschlusspaares bestimmt wird.

**4.** Verfahren nach Anspruch 3, wobei in Schritt (a) die Anschlusspaare so ausgewählt werden, dass jeder Kategorie mindestens ein gemessener Impedanzwert und/oder bestimmter vorkonditionierter Impedanzwert zugeordnet ist.

**5.** Verfahren nach Anspruch 3 oder 4, wobei das Verfahren ferner den Schritt umfasst, einen Durchschnittswert aus allen gemessenen Impedanzwerten und/oder bestimmten vorkonditionierten Impedanzwerten der jeweiligen Kategorie zu berechnen.

**6.** Verfahren nach einem der Ansprüche 3 bis 5, wobei das Verfahren ferner den Schritt umfasst, die genannten bestimmten durchschnittlichen gemessenen Impedanzwerte und/oder durchschnittlichen vorkonditionierten Impedanzwerte und/oder den gemessenen Impedanzwert und/oder die bestimmten vorkonditionierten Impedanzwerte über die jeweilige Kategorie in einem Diagramm abzutragen und ein Profil eines Graphen, der aus den genannten Werten über alle Kategorien in genanntem Diagramm gebildet ist, zu analysieren.

**7.** Verfahren nach einem der vorherigen Ansprüche, wobei das Verfahren ferner den Schritt umfasst, jeden gemessenen Impedanzwert und/oder vorkonditionierten Impedanzwert mit einem jeweiligen vordefinierten Zielwert für das jeweilige Anschlusspaar zu vergleichen, und/oder den Schritt, jeden durchschnittlichen gemessenen Impedanzwert und/oder durchschnittlichen vorkonditionierten Impedanzwert mit einem jeweiligen vordefinierten Ziel-Durchschnittswert für die jeweilige Kategorie zu vergleichen.

8. Verfahren nach Anspruch 6 oder 7, wobei die Art des Leitungskörpers (2,12) auf Basis des genannten Vergleichs und/oder des genannten Profils des Graphen in genanntem Diagramm identifiziert wird.

9. Verfahren nach Anspruch 7 oder 8, wobei das Verfahren ferner den Schritt umfasst, den einen Anschluss (10) der jeweiligen Anschlussgruppe (11), der elektrisch mit der am weitesten distalen Elektrode (4) der jeweiligen Elektrodengruppe (5) verbunden ist, auf Basis des genannten Vergleichs und/oder des genannten Profils des Graphen in genanntem Diagramm zu bestimmen.

10. Verfahren nach einem der vorstehenden Ansprüche, wobei die ausgewählten vordefinierten Anschlusspaare höchstens die Hälfte aller möglichen Anschlusspaare sind.

11. Verfahren für den Betrieb eines medizinischen Geräts, wobei das Verfahren die Schritte des Verfahrens zum Prüfen nach einem der Ansprüche 1 bis 10 umfasst und wobei das Verfahren ferner den Schritt umfasst, einen Betriebsmodus des Impulsgenerators (6) auf Basis des Verfahrens zum Prüfen auszuwählen, zum Beispiel auf Basis des identifizierten Typs von Leitungskörper (2), und dann nach dem ausgewählten Betriebsmodus funktioniert.

12. Medizinisches Gerät (1,1'), zum Beispiel zum Stimulieren des Rückenmarks, wobei genanntes medizinisches Gerät (1,1') mindestens zwei Elektrodengruppen (5) und einen Impulsgenerator (6) umfasst, wobei jede Elektrodengruppe (5) mindestens zwei Elektroden (4) umfasst, wobei die mindestens zwei Elektrodengruppen (5) auf einer Oberfläche (3) von mindestens einem Leitungskörper (2,12) angeordnet sind, wobei der Impulsgenerator (6) mindestens einen Port (9) umfasst, wobei die Anzahl der Anschlüsse (10) des mindestens einen Ports (9) gleich oder größer als die Anzahl der Elektroden (4) aller Elektrodengruppen (5) ist und Anschlussgruppen (11) bilden, die den Elektrodengruppen (5) entsprechen, wobei die Elektroden (4) elektrisch mittels mindestens eines Leitungsanschlusses (8,18) mit dem mindestens einen Port (9) des Impulsgenerators (6) verbunden sind, und wobei das medizinische Gerät (1,1') dafür konfiguriert ist, die Verfahrensschritte nach einem der Ansprüche 1 bis 11 auszuführen.

13. Medizinisches Gerät nach Anspruch 12, wobei der Leitungskörper (2,12) eine chirurgische Leitung oder eine perkutane Leitung ist.

14. System, das medizinische Gerät (1,1') von Anspruch 12 und einen Remote-Computer umfassend, wobei der Remote-Computer mindestens zeitweise mit dem medizinischen Gerät (1, 1') über eine Kommunikationsverbindung verbunden ist, und wobei der Remote-Computer dafür konfiguriert ist, einen Teil der Verfahrensschritte anstelle des medizinischen Geräts (1, 1') auszuführen.

15. Computerprogrammprodukt, das Anweisungen umfasst, die bei Ausführung einen Prozessor des medizinischen Geräts nach Anspruch 12 veranlassen, die Schritte der Verfahren nach einem der Ansprüche 1 bis 11 durchzuführen.

**Revendications**

1. Procédé de contrôle d'un dispositif médical (1,1'), dans lequel ledit dispositif médical (1,1') comprend au moins deux groupes d'électrodes (5) et un générateur d'impulsions (6), dans lequel chaque groupe d'électrodes (5) comprend au moins deux électrodes (4), dans lequel les au moins deux groupes d'électrodes (5) sont agencés sur une surface (3) d'au moins un corps de câble (2), dans lequel le générateur d'impulsions (6) comprend au moins un port (9), dans lequel le nombre de connexions (10) de l'au moins un port (9) est supérieur ou égal au nombre d'électrodes (4) de tous les groupes d'électrodes (5) et forme des groupes de connexions (11) correspondant aux groupes d'électrodes (5), dans lequel les électrodes (4) sont liées de manière électrique en utilisant au moins un connecteur de câble (8,18) à l'au moins un port (9) du générateur d'impulsions (6), et dans lequel le procédé comprend les étapes consistant à :

   (a) choisir des paires de connexions prédéfinies, dans lequel les connexions (10) de chaque paire sont attribuées à des groupes de connexions (11) différents, dans lequel au moins une connexion (10) est modifiée dans une paire prédéfinie par rapport à n'importe quelle autre paire choisie ;
   (b) mesurer une valeur d'impédance entre les connexions (10) de chacune des paires de connexions choisies ; et
   (c) contrôler si les liaisons électriques des électrodes (4) et les connexions (10) sont correctement établies en se basant sur des valeurs d'impédance préconditionnées déterminée et/ou les valeurs d'impédance mesurées des paires de connexion choisies, dans lequel la valeur d'impédance préconditionnée est déterminée pour chacune des paires de connexion choisies de telle sorte qu'elle fournit un préconditionnement de la valeur d'impédance mesurée correspondante pour atténuer le bruit indésirable.

**2.** Procédé selon la revendication 1, dans lequel le procédé comprend en outre l'étape consistant à étiqueter les connexions (10) d'au moins un port (9), dans lequel les connexions (10) d'un groupe de connexions (11) sont entièrement étiquetées d'une manière prédéfinie, par exemple numérotées de manière consécutive, dans lequel **l'étape** consistant à étiqueter toutes les connexions (10) est fournie de telle sorte qu'elle reflète la distance de chaque électrode (4) d'un groupe d'électrodes (5) spécifique à chaque électrode d'un groupe d'électrodes (5) différent si chaque électrode (4) est correctement liée électriquement à la connexion (10) correspondante.

**3.** Procédé selon la revendication 2, dans lequel le procédé comprend en outre l'étape consistant à attribuer chaque valeur d'impédance mesurée et/ou chaque valeur d'impédance préconditionnée d'une paire de connexions étiquetées à une catégorie d'un ensemble prédéfini de catégories, et dans lequel la catégorie d'une paire spécifique de connexions étiquetées est déterminée à partir des deux étiquettes de la paire de connexions spécifique.

**4.** Procédé selon la revendication 3, dans lequel dans l'étape (a) les paires de connexions sont choisies de telle sorte qu'au moins une valeur d'impédance mesurée et/ou une valeur d'impédance préconditionnée déterminée est attribuée à chaque catégorie.

**5.** Procédé selon la revendication 3 ou 4, dans lequel le procédé comprend en outre **l'étape** consistant à calculer une valeur moyenne pour toutes les valeurs d'impédance mesurées et/ou les valeurs d'impédance préconditionnées déterminées de la catégorie correspondante.

**6.** Procédé selon l'une quelconque des revendications 3 à 5, dans lequel le procédé comprend en outre l'étape consistant à tracer lesdites valeurs d'impédance mesurées moyennes et/ou les valeurs d'impédance préconditionnées moyennes et/ou la valeur d'impédance mesurée et/ou les valeurs d'impédance préconditionnées déterminées en fonction de la catégorie correspondante dans un diagramme et analyser un profil d'un graphique formé par lesdites valeurs en fonction de toutes les catégories dans ledit diagramme.

**7.** Procédé selon l'une quelconque des revendications précédentes, dans lequel le procédé comprend en outre l'étape consistant à comparer chaque valeur d'impédance mesurée et/ou valeur d'impédance préconditionnée à une valeur cible prédéfinie correspondante pour la paire de connexions correspondante et/ou l'étape consistant à comparer chaque valeur d'impédance mesurée moyenne et/ou valeur d'impédance préconditionnée moyenne à une valeur moyenne cible prédéfinie correspondante pour la catégorie correspondante.

**8.** Procédé selon la revendication 6 ou 7, dans lequel le type de corps de câble (2,12) est identifié en se basant sur ladite comparaison et/ou sur ledit profil du graphique dans ledit diagramme.

**9.** Procédé selon la revendication 7 ou 8, dans lequel le procédé comprend en outre **l'étape** consistant à déterminer la connexion (10) spécifique du groupe de connexions (11) correspondant lié de manière électrique à l'électrode (4) la plus distale du groupe d'électrodes (5) correspondant en se basant sur ladite comparaison et/ou sur ledit profil du graphique dans ledit diagramme.

**10.** Procédé selon l'une quelconque des revendications précédentes, dans lequel les paires de connexions prédéfinies choisies représentent au plus la moitié de toutes les paires de connexions possibles.

**11.** Procédé de fonctionnement d'un dispositif médical, dans lequel le procédé comprend les étapes du procédé de contrôle selon l'une quelconque des revendications 1 à 10, et dans lequel le procédé comprend en outre l'étape consistant à choisir un mode de fonctionnement du générateur d'impulsions (6) en se basant sur le procédé de contrôle, par exemple en se basant sur le type de corps de câble (2) identifié, puis fonctionne conformément au mode de fonctionnement choisi.

**12.** Dispositif médical (1,1'), par exemple pour stimulation de la moelle épinière, dans lequel ledit dispositif médical (1,1') comprend au moins deux groupes d'électrodes (5) et un générateur d'impulsions (6), dans lequel chaque groupe d'électrodes (5) comprend au moins deux électrodes (4), dans lequel les au moins deux groupes d'électrodes (5) sont agencés sur une surface (3) d'au moins un corps de câble (2,12), dans lequel le générateur d'impulsions (6) comprend au moins un port (9), dans lequel le nombre de connexions (10) de l'au moins un port (9) est supérieur ou égal au nombre d'électrodes (4) de tous les groupes d'électrodes (5) et forme des groupes de connexions (11) correspondant aux groupes d'électrodes (5), dans lequel les électrodes (4) sont liées de manière électrique en utilisant au moins un connecteur de câble (8,18) à l'au moins un port (9) du générateur d'impulsions (6), dans lequel le dispositif médical (1,1') est configuré pour exécuter les étapes de procédé selon l'une quelconque des revendications 1 à 11.

**13.** Dispositif médical selon la revendication 12, dans lequel le corps de câble (2,12) est un câble chirurgical ou un câble percutané.

**14.** Système comprenant le dispositif médical (1,1') selon la revendication 12 et un ordinateur distant, dans lequel l'ordinateur distant est au moins temporairement connecté au dispositif médical (1,1') par l'intermédiaire d'un lien de communication, et dans lequel l'ordinateur distant est configuré pour exécuter une partie des étapes de procédé à la place du dispositif médical (1,1').

**15.** Produit de programme informatique comprenant des instructions qui, lorsqu'elles sont exécutées, amènent un processeur du dispositif médical selon la revendication 12 à réaliser les étapes des procédés selon l'une quelconque des revendications 1 à 11.

FIG. 1

FIG. 2

EP 4 395 879 B1

FIG. 3

FIG. 4

FIG. 5

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200132434 A1
- US 20200093566 A1
- US 20190076659 A1